# EUROPÄISCHE PATENTSCHRIFT

(11) **EP 2 116 181 B1**
(45) Veröffentlichungstag und Bekanntmachung des Hinweises auf die Patenterteilung: **13.09.2017**
(21) Anmeldenummer: 08400056.1
(22) Anmeldetag: 22.12.2008
(51) Int. Cl.: A61B 5/00, A61B 5/087

(54) **Vorrichtung zur Bestimmung von Biodaten**
Device for recording biodata
Dispositif destinés à la détermination de données biologiques

(30) Priorität: 28.12.2007 DE 102007063554
(43) Veröffentlichungstag der Anmeldung: 11.11.2009
(73) Patentinhaber: Löwenstein Medical Technology S.A., 2557 Luxembourg (LU)
(72) Erfinder: Sommermeyer, Dirk, 76185 Karlsruhe (DE); Velykokhatko, Sergey, 76149 Karlsruhe (DE); Pulla, Matthias, 22459 Hamburg (DE); Wobig, Rüdiger, 76135 Karlsruhe (DE); Schöller, Bernd, 76135 Karlsruhe (DE)
(74) Vertreter: Patentanwälte Klickow & Partner Partnerschaftsgesellschaft mbB

(56) Entgegenhaltungen:
- WO-A-2004/032715
- WO-A-2007/083314
- WO-A-2007/115553
- US-A1- 2006 217 603

## Beschreibung

Die Erfindung betrifft eine Vorrichtung zum Messen von Biosignalen zur Erkennung schlafbezogener Atmungsstörungen gemäß des Anspruchs 1. Die Diagnostik von schlafbezogenen Atmungsstörungen (SBAS) erfolgt derzeit in Form einer Polysomnographie im Schlaflabor. Nach den Empfehlungen der Deutschen Gesellschaft für Schlafforschung und Schlafmedizin (DGSM) sind zur diagnostischen Messung mindestens 12 Registrierkanäle erforderlich. Bei der Polysomnographie im Schlaflabor werden unter Überwachungsbedingungen Körpersignale wie Sauerstoffsättigung, Atemfluss, EEG, EMG und EOG erfasst. Die Polysomnographie wird als Goldstandard der Diagnostik von SBAS angesehen.
Nach der Registrierung muss eine visuelle Auswertung des Schlafes und der Atmungsparameter zur Befunderstellung und diagnostischen Einordnung erfolgen. Dies setzt eine fundierte Kenntnis nicht nur der SBAS, sondern des gesamten Spektrums der Schlaf-Wach-Störungen voraus.
Aufgrund der erforderlichen hohen apparativen und personellen Ausstattung im Schlaflabor und der nach wie vor noch zu geringen Kapazitäten mit langen Wartezeiten für Patienten besteht ein Bedarf für ein einfaches Screening-System zu einem günstigen Preis, mit einfachster Handhabung, welches eine ambulante Diagnose ermöglicht. Dadurch wird das Screening für SBAS-Patienten in der Breite möglich und Risikopatienten werden frühzeitig entdeckt.

Aus der WO 2007/115553 A ist bereits eine Vorrichtung zur Messung von Biosignalen bekannt, die unter Verwendung mindestens einer Schnittstelle den Anschluss von Sensoreinrichtungen ermöglicht. Zur Anzeige von Messwerten ist eine Anzeigeeinrichtung sowie zur Funktionssteuerung eine Bedieneinrichtung vorgesehen.

In der WO 2004/032715 A wird ebenfalls eine Vorrichtung zum Messen von Biosignalen beschrieben, die mit einer Anzeigeeinrichtung und einer Bedieneinrichtung versehen sowie in einem Gehäuse angeordnet ist.

In der WO 2007/083314 A 1 wird ebenfalls eine Vorrichtung zum Messen von Biosignalen beschrieben, die in einem Gehäuse angeordnet ist und die sowohl eine Anzeigeeinrichtung als auch eine Bedieneinrichtung aufweist.

In der US 2006-217603 A1 werden Schnittstellen beschrieben, um eine Sensoreinrichtung an ein Gerätegehäuse anzukoppeln.

Aufgabe der vorliegenden Erfindung ist es, eine Vorrichtung der einleitend genannten Art derart zu verbessern, dass die Bestimmung von Biodaten erleichtert wird.

Diese Aufgabe wird erfindungsgemäß dadurch gelöst, dass die Vorrichtung dazu eingerichtet ist, über die zweite Schnittstelle mittels eines PC für eine automatische Messung derart konfiguriert zu werden, sich das Gerät automatisch zu einem konfigurierten Zeitpunkt einschaltet und mit der Messung beginnt.

Die beschriebene Vorrichtung stellt ein kleines Screeninggerät bereit, das mit den Parametern der Pulsoximetrie und des Flows (Nasenbrille) ein sicheres Screening für SBAS ermöglicht.

Die beschriebene Vorrichtung stellt darüber hinaus ein kleines Screeninggerät bereit, das mit den Parametern der Photoplethymographie und des Flows von Atemgas ein sicheres Screening für SBAS ermöglicht.

Die beschriebene Vorrichtung zeichnet diese Daten während ambulanter Messungen auf ein fest integriertes Speichermedium auf und zeigt ein (Teil-)Ergebnis sofort auf einem integrierten Display an. Die Daten werden über eine USB-Schnittstelle auf einen PC übertragen, wo mittels einer PC-Software die Rohdaten sichtbar und die Ereignisse editierbar sind und weitere Auswertungen durchgeführt werden können.

Für eine differenzierte Diagnose bietet die beschriebene Vorrichtung die Möglichkeit, einen echten Flow mittels Nasenbrille aufzuzeichnen. Neben Atemereignissen wie Apnoen oder Hypopnoen kann so auch die Flowkontur ausgewertet und z.B. Atemwegslimitationen diagnostiziert werden.

Durch eine Auswertung von wenigstens zwei der folgenden Signale ausgewählt aus der Gruppe: continous wave fluctuation: CWF, SpO2, Pulsfrequenz, Herzfrequenz, PTT (Puls-Transit-Zeit) beispielsweise durch Anwendung der Photoplethysmographie, ist es möglich, die Wechselwirkungen einer Vielzahl von physiologschen und pathophysiologischen Prozessen zu bestimmen. Zusätzlich ist es möglich, daß unter Verwendung einer gegenüber dem Stand der Technik erheblich reduzierten Anzahl von Messsensoren, nämlich nur einem Messsensor beispielsweise in Form eines PulsoximetrieSensors, vergleichbare Aussagen zu bereits bekannten Verfahren getroffen werden, beispielsweise hinsichtlich von Atmungsstörungen, Schlafstörungen, Diabetes, Entzündungseraktionen und Herz-Kreislauferkrenakungen.
Eine Möglichkeit zur Signalerfassung besteht darin, daß ein CWF (continous wave fluctuation)-Signal ausgewertet wird.
Dazu wird ein Plethysmogramm beispielsweise mit einem Pulsoximeter aufgezeichnet. Das Plethysmogramm unterliegt Schwankungen (= Fluktuationen), die für die Signalauswertung relevant sind. Aus dem Plethysmogramm wird ein CWF-Signal extrahiert. Das CWF-Signal enthält Informationen über die Schwankungen (= Fluktuationen) des Plethysmogramms. Beispielsweise kann die Schwankung die Pulswellenamplitude dargestellt werden. Es ist ebenfalls denkbar, das Integral des Plethysmogramms als CWF darzustellen. Es soll jegliche Schwankung des Plethysmogramms als CWF darstellbar sein.
Beispielsweise werden die Amplituden des Plethysmogramms zur Ermittlung des CWF-Signals verwendet. Das CWF stellt in diesem Fall die Amplitudenhöhe des Plethysmogramms dar.

Es ist jedoch ebenfalls daran gedacht, die PTT oder andere Signale, die Schwankungen unterliegen, zur Ermittlung einer CWF zu nutzen. Es können verschiedene Signale kombiniert werden, um relevante Schwankungen der Pulswelle zu detektieren und für die Auswertung heranzuziehen.
Eine andere Möglichkeit zur Signalerfassung besteht darin, daß ein Sp02-Signal ausgewertet wird.
Eine weitere Möglichkeit zur Signalerfassung besteht darin, daß ein Pulsfrequenz- und/oder Herzfrequenz-Signal ausgewertet wird.
Eine andere Möglichkeit zur Signalerfassung besteht darin, daß eine PTT (pulse transit time) ausgewertet wird.
Eine Signalauswertung über eine vorgebbare Zeitspanne wird dadurch unterstützt, daß der Sensor mit einer ersten Speichereinheit zur Abspeicherung eines erfaßten Meßsignals verbunden ist.
Insbesondere ist daran gedacht, daß vom Sensor die Variabilität der Herzfrequenz erfaßt wird.
Darüber hinaus ist es auch möglich, daß vom Sensor die PTT erfaßt wird.
Darüber hinaus ist es auch möglich, daß vom Sensor die CWF erfaßt wird.
Alternativ ist es möglich, daß vom Sensor die Pulsamplitude erfaßt wird.

Alternativ ist es möglich, daß vom Sensor ein Atemfluss erfaßt wird.

Die beschriebene Vorrichtung ermöglicht, in einem bevorzugten Ausführungsbeispiel, eine schnelle Ermittlung des Risiko-Index dadurch, dass mit Hilfe eines PulsoximetrieSensors zumindest drei Signale, nämlich CWF, Sauerstoffsättigung des Blutes und Pulsfrequenz, im wesentlichen gleichzeitig ermittelt werden. Durch eine Mustererkennung werden die Signale analysiert und mit gespeicherten Werten verglichen. Insbesondere wird die Veränderung der Amplitude der Pulswelle analysiert. Das Ergebnis des Vergleichs ergibt einen für den Patienten spezifischen Index-Wert, der geeignet ist, eine Aussage über das Risiko einer Herz-Kreislauferkrankung zu ermöglichen.
Um zukünftig auch eine drahtlose Datenübertragung (beispielsweise an ein Therapiegerät zur Druckregelung oder ein Handy zur Übertragung von Diagnoseparametern) zu ermöglichen, wird hardwaremäßig ein Bluetooth-Modul vorgesehen. Es ist vorgesehen den Direktdruck von Daten der Vorrichtung ohne den Einsatz eines PC durchzuführen. Hierzu wird die Vorrichtung über ein USB-Kabel direkt mit dem Drucker verbunden. In gleicher Weise kommuniziert die Vorrichtung mit anderen externen IT-Komponenten wie USB-Stick, USB-LAN-Konverter, USB-Bluetooth-Konverter etc.
Zum Transfer von Daten wird eine direkte Datenverbindung mit einem externen Gerät, insbesondere einem Drucker oder USB-Stick oder USB-LAN-Konverter oder USB-Bluetooth-Konverter, aufgebaut. Ein zwischengeschalteter PC ist dazu unnötig.
Alternativ könnte auch eine andere Verbindungsmöglichkeiten wie z. B. Bluetooth genutzt werden. Damit das direkte Drukken funktioniert, weisen die Vorrichtung und der angeschlossene Drucker einen gemeinsamen Kommunikationsstandard auf und sind beispielsweise über ein USB-Kabel miteinander verbunden. Über die Benutzeroberfläche der erfindungsgemäßen Vorrichtung kann dann der Ausdruck gestartet werden. Alternativ ist auch vorgesehen, dass der Ausdruck über das Druckermenue gesteuert werden kann. Ziele:
∘ Zuverlässige Aussagen über das Bestehen einer schlafbezogenen Atmungsstörung (SBAS) bei einfachster Applikation.
∘ Differenzierung der SBAS in obstruktives bzw. zentrales Leiden.
∘ Risikoeinschätzung, ob eine PLM-Erkrankung vorliegt.
∘ Optionale Möglichkeit eine Nasenbrille anzuschließen.
Die beschriebene Vorrichtung wird am Handgelenk wie eine Uhr getragen. Die Bedienung erfolgt mit einer Taste. Zur Applikationshilfe werden die Anschlüsse mit aussagekräftigen Symbolen versehen.
Als Sensoren sind vorgesehen:
∘ Pulsoximetriesensor zur Erfassung von Sauerstoffsättigung, Pulsfrequenz und Pulswelle / alternativ Photoplethysmographie-Sensor
∘ Atemfluss-Schnarch-Nasenbrille (Drucksensor)
Die Datenübertragung erfolgt über eine USB-Schnittstelle. Das Gerät wird hierzu vom Patienten abgenommen (Verzicht auf galvanische Trennung). Die beschriebene Vorrichtung ist so konstruiert, dass eine Datenübertragung nicht möglich ist, wenn der Patient das Gerät appliziert hat.

Die beschriebene Vorrichtung wird über handelsübliche Akkus oder Batterien mit Strom versorgt.
Die im Gerät gespeicherten Daten können an einen PC übertragen werden. In diesem Fall kann der Anwender Rohdaten und Analyseergebnisse einsehen, Ereignisse und Analysebericht editieren/konfigurieren, einen automatisch generierten Bericht ausdrucken, ohne dass ein manuelles Auswerten erforderlich ist.
Das Diagnosegerät wird über eine PC-Software konfiguriert. Hierzu wird die SOMNOlab Software verwendet.
Die automatischen Analysen (Atmungsanalyse, Pulswellenanalyse) erfolgen online im Gerät, um dem Anwender nach Beenden der Messung direkt ein Ergebnis anzuzeigen. Die Analysen unterstützen den Auswerter bei der Diagnose von Schlafstörungen, sowie der Therapieeinleitung und Therapiekontrolle.
Bezüglich der elektrischen Sicherheit gehören PC-Systeme nicht zum beschriebenen Vorrichtung-Anwendungsteil. Das verwendete PC-System muss die Richtlinien der EN 60950 erfüllen.
Die PC-Software dient der Visualisierung, Auswertung, Dokumentation und patientenbezogenen Archivierung von Langzeituntersuchungen zur Diagnostik von Schlafstörungen. Die Software erlaubt die Eingabe von Bemerkungen durch den Anwender. Eine manuelle Reklassifikation der Analyseergebnisse durch den Auswerter ist möglich.
Neben der Uhrzeit werden der voraussichtliche Messbeginn und die Anzeige (Laie / Experte) für den Offline-Fall über den PC konfiguriert.
Vorgesehen ist das System für Personen ab 2 kg Körpergewicht unter Verwendung der spezifizierten Sensoren. Bei den Analysen sind keine Beeinträchtigungen bezüglich der Respiration bekannt.
Das Fachpersonal bereitet die Messung vor, bedient die Software und versorgt das Gerät mit Akkus/Batterien.
Der Patient ist nach Einweisung durch das Fachpersonal und anhand der Gebrauchsanweisung für den Patienten in der Lage, die Sensoren und das Gerät selbst anzulegen.

Die beschriebene Vorrichtung ist ein Datenaufzeichnungssystem zur Erfassung, Aufzeichnung, Abspeicherung und Auswertung von Biosignalen während des Schlafes. Es dient der Erkennung schlafbezogener Atmungsstörungen und begleitender Risikofaktoren zur Unterstützung der Diagnose, sowie der Therapieeinstellung und Therapiekontrolle. Einsatzgebiete sind Untersuchungen beim Patienten zu Hause oder im klinischen Bereich.
Die gemessenen Daten werden im Gerät gespeichert. Analyseergebnisse werden auf einem Gerätedisplay angezeigt. Gespeicherte Daten können über eine USB-Schnittstelle an den PC übertragen werden.

Die mitgelieferte PC-Software dient der Speicherung, Verarbeitung, Visualisierung, Auswertung, Dokumentation und der Archivierung der von der beschriebenen Vorrichtung gemessenen Daten.

Die beschriebene Vorrichtung erfasst folgende schlafbezogene Parameter:
∘ Sauerstoffsättigung (SpO2; pulsoximetrisch)
∘ Pulsfrequenz (pulsoximetrisch)
∘ Roh-Pulswelle
∘ Qualitätsindex der Sauerstoffsättigung
∘ Nasaler Atemflow (optional)
∘ Schnarchen (optional)

Somit ist die vorliegende Vorrichtung das einzige Screeninggerät mit miniaturisierter Pulsoximetrie und integrierter Flow-Messung mit Angaben zum obstruktiven oder zentralen Charakter von SBAS.
PC unabhängige Analysen sowie das Anzeigen der Analyseergebnisse erfolgen auf dem integrierten Display, wodurch eine Zeitersparnis für den Arzt erreicht wird.

Einsatzgebiet für die beschriebene Vorrichtung ist die erste Abklärung eines Verdachts auf SBAS sowie - in bestimmten Bereichen - das Screening nach SBAS.

Die beschriebene Vorrichtung stellt ein kleines Screeninggerät bereit, das mittels Pulsoximetersensor (am Finger) die Signale Sp02, Pulsfrequenz und Pulswelle aufzeichnet, sowie optional mittels Staudruckmessung (Nasenbrillen-Flow-Sensor) ein Atemfluss-Signal speichern kann.
Die Signale werden stets im Gerät gespeichert.

Das integrierte Display stellt nach erfolgter Messung verschiedene, einfach zu verstehende Ergebnisparameter dar, z.B. einen Risiko-Wert für eine vorhandene SBAS (z.B. auch als Balken visualisiert), ggf. auch den berechneten AHI, usw.
Die Rohdaten können anschließend über eine Schnittstelle, beispielsweise eine USB-Schnittstelle, auf einen PC übertragen werden, wo mittels einer PC-Software die Signale sicht- und editierbar sind, weitere Analysen und Auswertungen durchgeführt werden können und ein entsprechender Bericht generiert werden kann. Atemflow und Schnarchen via Atemfluss-Schnarch-Nasenbrille. Sauerstoffsättigung / Pulsfrequenz / Pulswelle über Pulsoximetrie.

Die Anbringung der beschriebenen Vorrichtung am Patienten erfolgt am Handgelenk. Dazu weist die Gehäuseunterseite einen Krümmungsradius auf, der der Armoberfläche im Bereich des Unterarmes / des Handgelenks angepasst ist. Die Daten werden gespeichert und offline per USB-Kabel an den PC übertragen. Die Stromversorgung erfolgt über 2 AA-Batterien oder 2 AA Akkus.
Pulsoximeter: Anschluss direkt am Gerät mit platzsparender Steckverbindung. Pulsoximeter mit Gummisensor für den Finger Atemfluss-Schnarch-Nasenbrille, Flowsensor mit Nasenbrille.
Die Befestigung der Vorrichtung am Unterarm / Handgelenk hat den Vorteil, dass ein kurzer Weg zum Fingerclip-Sensor besteht.
Display
o Gerät mit Display (Kurvendarstellung und drei Zahlen)
o Display ca 35mm x 35mm oder 30mm x 40mm Sichtbarer Bereich 28mm x 28mm

Im Betriebsmodus Online erfolgt die Anzeige der aktuellen Werte für:
∘ Sauerstoffsättigung
∘ Puls
∘ Flow
∘ Plethysmogram Blutdruckkurve

Im Betriebsmodus Offline erfolgt die Anzeige der Ergebnisse:
∘ AHI obstructive (/h)
∘ AHI central (/h)
∘ Desaturation index (/h)
∘ PWA result (attenuations?)
∘ Autonomous reaction (sleep Quality)
∘ Arousals with/without resp. event
∘ Snoring events and in/expiration
∘ Flattening events and in/expiration

Bedienelement: Ein Bedienknopf
∘ 4 Werte beim Messen
∘ 8 Werte zum Auslesen
∘ 4 max. 5 Werte für den Patienten

### PC-Software:

Die im Gerät gespeicherten Daten können an einen PC übertragen werden. In diesem Fall kann der Anwender Rohdaten und Analyseergebnisse einsehen, Ereignisse und Analysebericht editieren/konfigurieren, sowie einen automatisch generierten Bericht, ohne manuelles Auswerten ausdrucken.

Das Diagnosegerät wird über eine PC-Software konfiguriert. Hierzu wird die SOMNOlab Software verwendet. Das Aktivieren und Deaktivieren der beschriebenen Vorrichtung erfolgt durch Setzen eines Häkchens im Optionen Dialog. Wenn nur die beschriebene Vorrichtung aktiviert ist, reduzieren sich die Funktionalitäten auf ein Minimum. Der Reporter der beschriebenen Vorrichtung entfällt.
∘ PC Software unabhängige Analysen, integriert im Gerät
∘ Analysen laufen online im Gerät
∘ Analyseergebnisse werden direkt bei Beenden der Messung auf (O-LED-)Display angezeigt
∘ Online Anzeige der aktuellen Messwerte (Pulsoximeter, Flow)
∘ Angabe von Risikofaktoren für einzelne Krankheitsbilder (z.B. OSAS) werden farblich auf dem Display dargestellt (z.B. von grün nach rot)
∘ Signalanalyse, Signalverarbeitung mit Fixpoint Arithmetik (Filterung)
∘ Schlafscreening am Handgelenk
∘ Armband mit Doppelschlaufe
∘ Die Applikation des Gerätes ist derart variabel, dass man es rechts und links am Arm tragen kann. Dabei kann auch die Anzeige auf dem Display um 180° gedreht werden.
∘ Datenübertragung: Online drahtlos mit Bluetooth und USB:
   Ereignisse werden an Server übertragen
∘ Datenübertragung Ereignisse drahtlos an Handy und dann weiter über Internet
∘ Einknopfbedienung
∘ Optimierung des Flow-Signals, Signalqualität bei z.B. schlechter Applikation durch automatische Verstärkungsanpas-sung des Flow-Signals
∘ Die Sprache ist am Display konfigurierbar

Die Schlafdiagnose ist durch ein Gerät am Handgelenk OHNE PC möglich.

Analyse von schlafbezogenen Atmungsstörungen - differenziert nach obstruktiv/zentral - mit Anzeige der Ergebnisse auf dem Display, OHNE dass das Gerät mit dem PC verbunden werden muss und Daten übertragen werden müssen. Das ist zwar auch mög-lich, aber nicht zwingend notwendig.

Die USB-Buchse für die mögliche Datenübertragung wird am Ge-rät so vorgesehen, dass allein durch die Lage der Buchse ver-hindert wird, dass ein USB-Stecker angeschlossen werden kann, wenn das Gerät gerade am Patienten appliziert ist.
a. online Analyse der Ereignisse und Anzeige der Werte, z.b. AHI , durch Matching pursuit Verfahren
   Schneller AM7 µC, Signalanalyse erfolgt mit Fixpunkt Arithmetik, angepasst auf µC
   Daten werden im Speicher abgelegt und gleichzeitig werden die letzten 10 min analysiert
   Ereignisse werden drahtlos an Server übertragen
   Ereignisse werden über 10 Nächte im Gerät gespeichert
b. kann PC unabhängig betrieben werden
c. Risikofaktoren werden ermittelt und auf Display farblich hervorgehoben
d. Matching pursuit Signalverarbeitung
e. Gerät wird auf Armband mittels einer Doppelschlaufe aufgeklickt. Vorteil: USB-Schnittstelle ist unter dem Gerät. Dadurch besteht im Betrieb keine Mölichkeit für einen Kontakt zum Patienten -> die galvanische Trennung für die USB-Schnittstelle entfällt
f. Beim Einstecken von USB-Kabel wechselt Gerät automatisch in PC-Modus
g. Einknopfbedienung
h. Einstellbar: Gerät schaltet sich immer zur gleichen Zeit für Messungen ein
i. Wechsel zwischen online-Modus (Messen) und offline-Modus (Analyse) über Erkennung der Sensorbelegung: Finger drin = online / Finger draußen = offline
j. Automatische Nullpunktkalibrierung für Drucksensor -> wartungsarm (Kalibrierung erfolgt wie bei Küchenwaage)

In den Zeichnungen sind Ausführungsbeispiele der beschriebenen Vorrichtung schematisch dargestellt. Es zeigen:
- Fig. 1: Eine perspektivische Darstellung eines Armbandes mit einer abnehmbaren Vorrichtung zur Messung von Biosignalen,
- Fig. 2: die Anordnung gemäß Fig. 1 nach einer Trennung des Armbandes und der Meßvorrichtung,
- Fig. 3: die Anordnung gemäß Fig. 2 bei einer Blickrichtung von unten,
- Fig. 4: die Anordnung gemäß Fig. 3 nach einem Abnehmen eines Deckels eines Batteriefaches und
- Fig. 5: eine schematische Darstellung der verwendeten Funktionskomponenten.

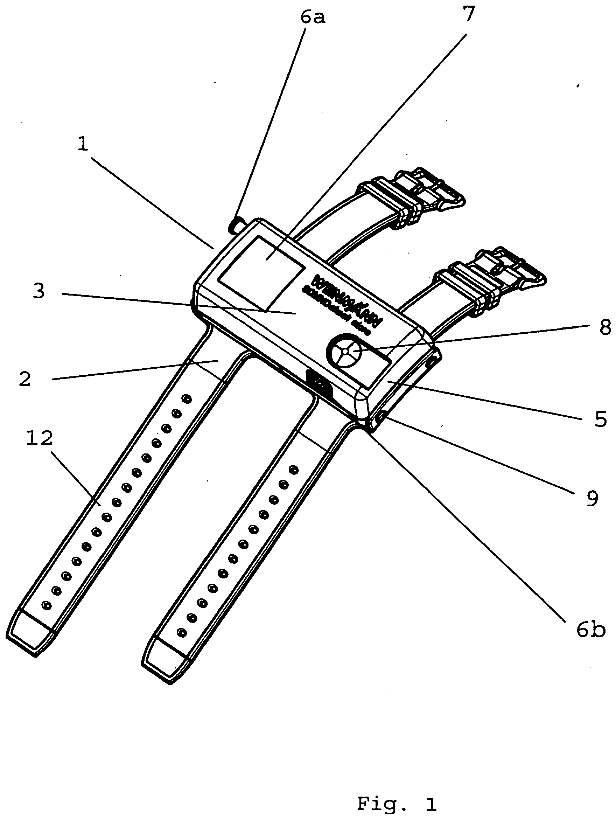

Fig. 1 zeigt in perspektivischer Darstellung das Armband (2) mit Laschen (12), welches der Befestigung am Körper eines Patienten dient, und die auf dem Armband abnehmbar montierte Vorrichtung zur Messung von Biosignalen (1). Die Vorrichtung zum Messen von Biosignalen weist ein Gehäuse (1) auf, welches aus einer Oberseite (3) und einer Unterseite (4) und Seitenteilbereichen (5) besteht. In einem ersten Seitenteilbereich (5) des Gehäuses befindet sich eine erste Schnittstelle (6a) zum Anschluß der Flow/Druck Sensoreinrichtung. In einem zweiten Seitenteilbereich (5) des Gehäuses befindet sich eine zweite Schnittstelle (6b), die dem Anschluß eines Datenkabels oder dem Anschluß eines Pulsoximetrie-Sensors oder dem Anschluß eines Photoplethymographie-Sensors dient. Eine Anzeigeeinrichtung (7), hier als Display ausgeführt, befindet sich im Bereich einer Oberseite (3) des Gehäuses (1) und dient zur Anzeige von Meßwerten und Statusinformationen.

Eine Bedieneinrichtung (8) befindet sich ebenfalls im Bereich einer Oberseite (3) des Gehäuses (1) und dient zur Funktionssteuerung der Vorrichtung. Die Bedieneinrichtung (8) ist als drückbare Taste ausgeführt und dient dem Ein- und Ausschalten, sowie der Navigation im Gerätemenue, welches über das Display dargestellt wird. Die Auswahl eines Menuepunktes erfoglt dabei durch ein doppeltes Drücken der Bedieneinrichtung (8). Ein- und Ausschalten erfolgt durch einmaliges Drücken für zumindest drei Sekunden. Befestigungselemente (9) im Seitenteilbereich des Gehäuses (1) greifen in einem montierten Zustand in Ausnehmungen (10) der Befestigungseinrichtung (2) ein und fixieren somit das Gehäuse auf dem Armband.

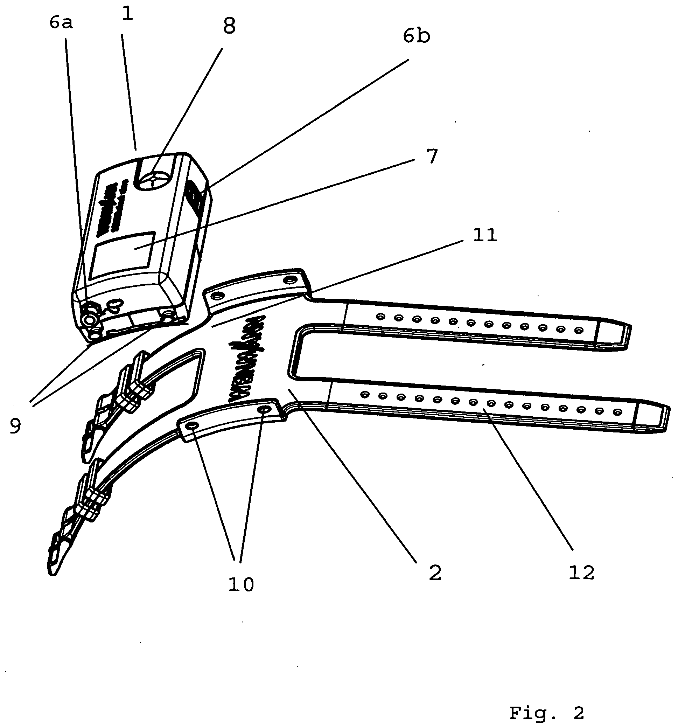

Fig. 2 zeigt die Anordnung gemäß Fig. 1 nach einer Trennung des Armbandes (2) und der Meß-vorrichtung (1). Es ist erkennbar, dass das Gehäuse (1) lösbar mit der Befestigungseinrichtung (2) verbunden ist. Dazu weist das Gehäuse Befestigungselemente (9) auf, die als Zapfen (9) im Seitenteilbereich des Gehäuses ausgebildet sind und die in einem montierten Zustand in Ausnehmungen (10) der Befestigungseinrichtung (2) eingreifen. Die Befestigungseinrichtung (2) weist eine Aufnahmefläche (11) für das Gehäuse auf. Die Aufnahmefläche bildet einen zentralen plattenartigen Bereich aus, von dem Laschen (12) abgehen, die der Befestigung an einem Körperteil (beispielsweise Handgelenk) dienen.

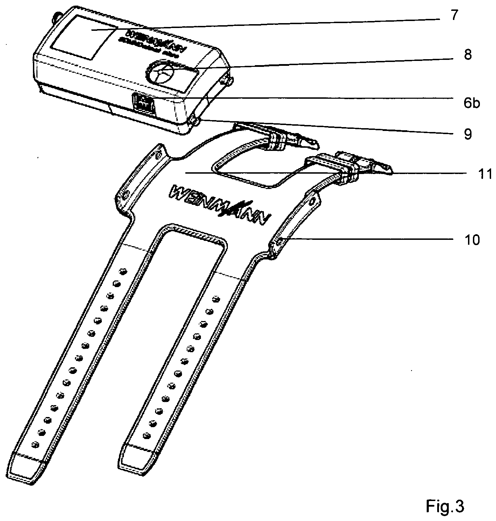

Fig. 3 zeigt die Anordnung gemäß Fig. 2 bei einer Blickrichtung von unten. Im Bereich der Unterseite (4) des Gehäuses ist eine USB-Schnittstelle (13) angeordnet, die mit einem Deckel (14) verschließbar ist. Die USB-Schnittstelle (13) ist nach einer Montage des Gehäuses im Bereich der Befestigungseinrichtung (2) von der Befestigungseinrichtung (2) abgedeckt. Die Befestigungseinrichtung (2) ist zumindest an dieser Stelle elektrisch nicht leitend ausgeführt. Somit besteht kein Kontakt, insbesondere kein elektrisch leitender Kontakt, zum Patienten. Daher kann auf eine galvanische Trennung der USB-Schnittstelle verzichtet werden. Die USB-Schnittstelle (13) ist derart angeordnet und isoliert, dass im Betrieb der Vorrichtung ein Kontakt zwischen einem Nutzer und der USB-Schnittstelle ausgeschlossen ist.

Weiterhin befindet sich im Bereich der Unterseite (4) des Gehäuses ein Batteriefach, das mit einem Deckel (14) verschlossen ist.

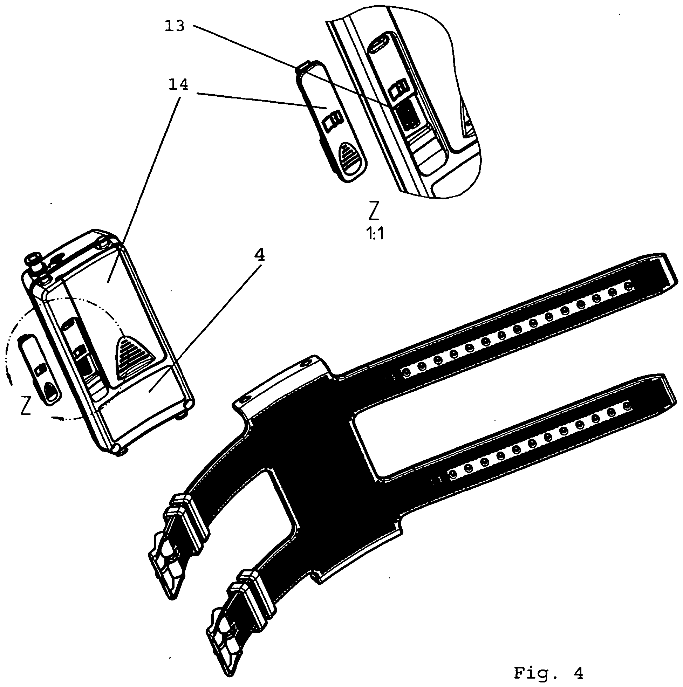

Fig. 4 zeigt die Anordnung gemäß Fig. 3. Man erkennt neben der USB-Schnittstelle, deren Deckel (14) hier abgenommen ist, auch das Batteriefach (15), ebenfalls mit abgenommem Deckel (14). Im Batteriefach befinden sich zwei Batterien oder Akkumulatoren, die die Energieversorgung für mindestens 8 h aufrecht erhalten.

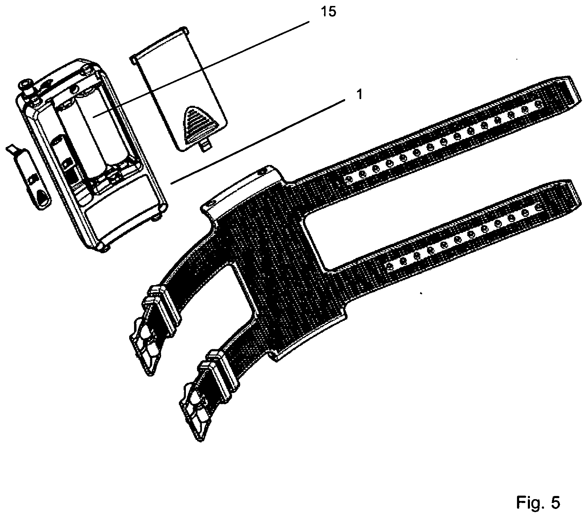

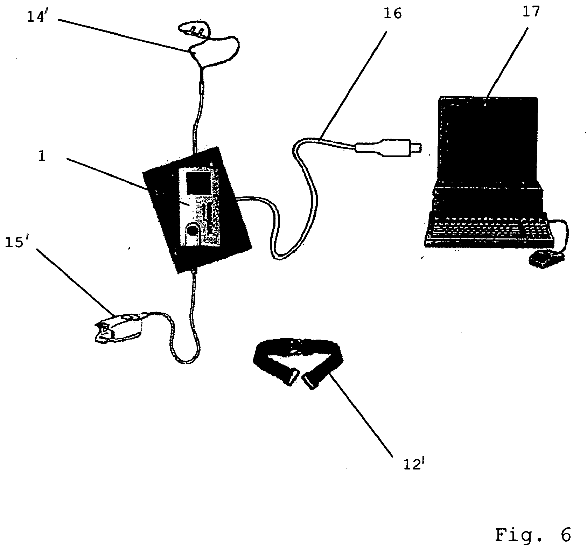

Fig. 5 zeigt eine schematische Darstellung der verwendeten Funktionskomponenten und veranschaulicht die Funktionsweise des Gesamtsystems. Das Gerät (1) ist ein Datenaufzeichnungssystem zur Erfassung, Aufzeichnung, Abspeicherung und Auswertung von Biosignalen während des Schlafes. Es dient der Erkennung schlafbezogener Atmungsstörungen und begleitender Risikofaktoren zur Unterstützung der Diagnose sowie der Therapieeinstellung und Therapiekontrolle. Einsatzgebiete sind ambulante Untersuchungen beim Patienten zu Hause oder im klinischen Bereich. Eine Einweisung des Patienten hinsichtlich Anwendung und Funktionen des Gerätes erfolgt durch den Arzt und das vom Arzt eingewiesene Fachpersonal. Die gemessenen Daten werden im Gerät gespeichert. Analyseergebnisse werden auf einem Gerätedisplay angezeigt. Gespeicherte Daten können über eine USB-Schnittstelle (16) an den PC (17) übertragen werden. Die mitgelieferte PC-Software dient der Speicherung, Verarbeitung, Visualisierung, Auswertung, Dokumentation und Archivierung der gemessenen Daten.
Das Gerät erfasst folgende schlafbezogene Parameter:
∘Sauerstoffsättigung (Sp02; pulsoximetrisch)
∘Pulsfrequenz (pulsoximetrisch)
∘Rohpulswelle
∘Qualitätsindex der Sauerstoffsättigung
∘Nasaler Atemflow (optional)
∘Schnarchen (optional)
Das Geräte-System besteht aus folgenden Komponenten:
- Screening-Gerät (1)
- Pulsoximetriesensor (15')
- Staudruck-Nasenbrille (14')
- Armband (12')
- Software (17) auf einem PC laufend

Zentraler Bestandteil des Systems ist das kleine Screening-Gerät (1), das mit einem Armband am Handgelenk des Patienten angebracht wird. An den Zeigefinger des Patienten wird ein Pulsoximetriesensor (15') angelegt. Dieser Pulsoximetriesensor misst die Sauerstoffsättigung (Sp02), die Pulsfrequenz und die Pulswelle. Optional erfasst eine Staudruck-Nasenbrille (14') den Atemfluss und das Schnarchen des Patienten. Alle Signale werden im Gerät (1) gespeichert. Zur Verarbeitung von Messinformationen weist das Gerät eine Recheneinheit auf, die in dem Gehäuse (1) zur Verarbeitung der Messsignale angeordnet ist, und einen Speicher auf, der mit der Recheneinheit zum Anzeigen und/oder Speichern der Messinformationen in Verbindung steht. Die Daten werden im Speicher abgelegt und gleichzeitig werden die letzten 10 Minuten der Daten analysiert. Zur Signalanalyse werden eine Fixpunkt-Arithmetik und/oder ein Matching pursuit Verfahren eingesetzt. Die Messdaten werden während einer Messung auf ein Speichermedium geschrieben und parallel zumindest teilweise auf dem Display angezeigt.
Die im Gerät gespeicherten Daten und Ergebnisse werden über eine USB-Verbindungsleitung an einen PC übertragen. In der Software können die Rohdaten und Analyseergebnisse eingesehen, Ereignisse und Analyseberichte editiert und konfiguriert sowie ein automatisch generierter Bericht ausgedruckt werden. Mit Hilfe der vom Gerät übertragenen Analyseergebnisse und der in der Software dargestellten Signale können die Ergebnisse vom Arzt bewertet werden.

Das Gerät wird mit einer Taste eingeschaltet und erfasst nur mit Hilfe der Staudruck-Nasenbrille und des Pulsoximetriesensors schlafbezogene Parameter. Diese Ergebnisparameter werden nach erfolgter Messung auf dem Display dargestellt. Für eine Messung stehen drei Modi zur Verfügung, die über den PC konfiguriert werden können:

Online-Modus: In diesem Modus werden die aktuell gemessenen Signale dargestellt. Empfängt das Gerät außerhalb der programmierten Messzeit ein gültiges Signal, wechselt das Gerät vom Offline-Modus in den Online-Modus. Das Gerät zeigt die aktuellen Messwerte auf dem Display an, speichert sie jedoch nicht. Empfängt Das Gerät keine Signale mehr, wechselt das Gerät automatisch wieder in den Offline-Modus. Auf dem Display werden die Ergebnisse der letzten gespeicherten Messung angezeigt. Wird bei ausgeschaltetem Display die Taste an Das Gerät gedrückt, schaltet sich nur das Display an. Wiederholtes Drücken der Taste zeigt die einzelnen Ergebnisse der letzten Messung an. Empfängt Das Gerät innerhalb der programmierten Messzeit mindestens ein gültiges Signal, zeigt das Gerät die aktuellen Messwerte auf dem Display an und speichert diese. Empfängt Das Gerät keine Signale mehr, speichert das Gerät bis zum Erreichen der programmierten Endzeit Nullwerte und schaltet sich dann automatisch aus. Je nach angelegtem Sensor bzw. empfangenen Signalen können folgende aktuelle Messwerte angezeigt werden:

**Tabelle 1**

| **Sensor** | **Online**-**Messwerte** |
|---|---|
| **Nur Pulsoximeter** | - SpO2 |
| | - Pulsfrequenz |
| **Nur Staudruck-Nasenbrille** | - Atemfluss |
| | - Pulsfrequenz |
| | - Atemfluss |
| **Beide** | - SpO2 |

**Offline-Modus:** Das Gerät befindet sich im Offline-Modus, wenn das Gerät durch Drücken auf die Taste eingeschaltet wird und keine Signale anliegen. Auf dem Display werden die Ergebnisse der letzten gespeicherten Messung angezeigt. Wiederholtes Drücken der Taste zeigt die einzelnen Ergebnisse der letzten Messung an. Je nach angelegtem Sensor und konfigurierter Anzeige können folgende Ergebnisse angezeigt werden:

**Tabelle 2**

| **Sensor** | **Offline-Messwerte** |
|---|---|
| **Nur Pulsoximeter** | - Obstruktives Schlafapnoe-Risiko |
| | - Zentrales Schlafapnoe-Risiko |
| | - RDI, oRDI, zRDI |
| | - ODI |
| | - Durchschnittlicher SpO2/minimaler Sp02 |
| | - Zeit unter x % Sp02 |
| | - Pulsfrequenzvariationsindex |
| | - Durchschnittliche Pulsfrequenz |
| | - RDI (mit autonomem Arousal) |
| | - Autonomer Arousal-Index (ohne RD) |
| **Nur Staudruck-Nasenbrille** | - Schlafapnoe-Risiko |
| | - AHI |
| | - Schnarchanteil |
| | - Flatteninganteil |
| **Beide** | - Obstruktives Schlafapnoe-Risiko |
| | - Zentrales Schlafapnoe-Risiko |
| | - AHI, oAHI, zAHI |
| | - Schnarchanteil |
| | - Flatteninganteil |
| | - ODI |
| | - Durchschnittlicher SpO2/minimaler Sp02 |
| | - Zeit unter x % Sp02 |
| | - PFV-Index |
| | - Durchschnittliche Pulsfrequenz |
| | - RDI (mit autonomem Arousal) |
| | - Autonomer Arousal-Index (ohne RD) |
| | - Flattening-Index |
| | - Autonome Arousals mit Flattening |

Zumindest zwei Betriebszustände werden automatisch durchgeführt, nämlich
a) der Betriebszustand online, durch Einlegen eines Körperteils in den angeschlossenen Pulsoximetriesensor und
b) der Betriebszustand offline, durch Herausnehmen eines Körperteils aus dem angeschlossenen Pulsoximetriesensor.
Im Betriebszustand online erfolgt eine Registrierung von Biosignalen und im Betriebszustand offline eine Analyse der Biosignal-Messwerte.

PC-Modus: Das Gerät schaltet automatisch in den PC-Modus, sobald das Gerät über eine USB-Verbindungsleitung mit einem PC verbunden wird. Die aktuell gespeicherte Messung und alle bisher noch nicht übertragenen Auswerteergebnisse werden in die Software importiert. Die Messdaten werden über eine USB-Schnittstelle auf einen PC übertragen, wenn durch Einstecken des USB-Steckers in die USB-Schnittstelle automatisch der Betriebszustand PC-Modus geschaltet wird, der eine Übertragung der Daten ermöglicht. Die Datenübertragung über eine USB-Schnittstelle erfolgt nur dann, wenn der Patient die Vorrichtung zur Aufnahme von Biosignalen nicht appliziert hat.

Die von Das Gerät erfassten Daten werden nach einer Messung in die PC-Software SOMNOlab importiert.

SOMNOlab speichert, visualisiert und wertet die importierten Daten aus und archiviert diese anschließend.
SOMNOlab bietet für Das Gerät zwei Modi:
∘ Expressmodus: Der Expressmodus ermöglicht nur einen Lesezugriff auf die Messmodus-Konfigurationen.
   Der Messmodus-Dialog ist stark vereinfacht.
∘ Expertenmodus: Im Expertenmodus können Ereignisse editiert und unterschiedliche Konfigurationen gewählt werden.

Der Pulsoximetriesensor erfasst die pulsoximetrischen Signale, d.h. die Sauerstoffsättigung des Blutes und die Pulsfrequenz des Patienten. Die Hauptbestandteile des Pulsoximetriesensors sind zwei Leuchtdioden und eine Empfängerdiode. Für jede Pulswelle werden mehrere Sp02-Werte bestimmt (Split-Pulswave-Algorithmus). Das Gerät errechnet zu jedem erfassten Sauerstoffsättigungswert einen Qualitätsindex, welcher die Güte bzw. die Genauigkeit des gemessenen Sp02-Wertes kennzeichnet. Wird das Signal bei Bewegungen gestört, ist die Anzahl verwertbarer Messwerte geringer. Bei störungsfreien Signalen liegt einen hohe Anzahl von Werten vor. Dementsprechend erzeugt ein gestörtes Messsignal einen niedrigen Qualitätswert, ein ungestörtes Messsignal hat einen hohen Qualitätswert zur Folge. Das Qualitätssignal nimmt Werte zwischen 0% und 100% an. Bei der Beurteilung von Sp02-Langzeitmessungen kann das Qualitätssignal hilfreich sein, denn eslässt auf Artefakte schließen, die während der Messung auftraten.

Der Pulsoximetriesensor ist durch Referenzmessungen mittels fraktioneller Sättigungsmessung auf die pulsoximetrische Hämoglobin-Sauerstoffsättigung bei dishämoglobinfreiem Blut kalibriert. Mit Hilfe des Pulsoximetriesensors wird am Finger des Patienten, also nicht-invasiv, die funktionelle Sauerstoffsättigung des arteriellen Blutes bestimmt. Ist der Anteil an disfunktionellem Hämoglobin (z.B. Carboxihämoglobin oder Methämoglobin) hoch, wird die Genauigkeit der Messung beeinträchtigt. Störungen im Messsignal werden vom Gerät überwacht. Das Gerät liefert Fehlermeldungen, wenn die Störungen außerhalb der intern festgelegten Grenzen liegen. Das Gerät schränkt dann seinen Messbetrieb ein. Es werden "0"-Werte angezeigt. Normales Umgebungslicht wird vom Sensor kompensiert. Besonders starkes oder schwankendes Umgebungslicht, z.B. durch direkte Sonneneinstrahlung oder OP-Lampen, kann die Messergebnisse verfälschen. Das Gerät schränkt dann seinen Messbetrieb ein. Es werden "0"-Werte angezeigt. Das Gerät erkennt Bewegungsartefakte, unterdrückt diese weitestgehend mit Hilfe von verschiedenen Algorithmen und meldet, sobald sie zu hoch werden.

Die Staudruck-Nasenbrille dient der Diagnosemessung.
Die Staudruck-Nasenbrille erfasst in Verbindung mit dem in Das Gerät integrierten Drucksensor den Atemfluss sowie das Schnarchen. Die Inspiration wird über den erzeugten Unterdruck registriert, die Exspiration über den erzeugten Überdruck. Schnarchen erzeugt Druchschwankungen in den Nasenöffnungen, die ebenfalls registriert werden. Die Druckmessung reagiert bei geschlossenem Mund sensibler auf geringe Flusslimitierungen als die thermische Messung. Sie ist unabhängig von der Umgebungstemperatur und ermöglicht zusätzlich die visuelle Beurteilung der zeitlichen Flusskontur. Die Signaldarstellung sowie die automatische Analyse sind auf die original WEINMANN Staudruck-Nasenbrille abgestimmt. Es wird die Differenz zwischen nasalem Druck und Umgebungsdruck erfasst. Die Schnarcherkennung erfolgt aus der Staudruck-Nasenbrille.

Um das Gerät zu konfigurieren oder die in dem Gerät gespeicherten Daten an Ihren PC zu übertragen, muss das Gerät über eine USB-Verbindungsleitung mit dem PC verbunden werden.

Bei einer automatischen Messung schaltet sich das Gerät erfindungsgemäß automatisch zum konfigurierten Zeitpunkt ein und beginnt mit der Messung. Um eine manuelle Messung zu starten, muss die Taste (8) an dem Gerät 3 Sekunden lang gedrückt gehalten werden. Das Gerät schaltet sich dann ein und beginnt mit der Messung.
Um in der Software eine automatische Messung durchzuführen, wird ein Startzeitpunkt festgelegt. Das Gerät schaltet sich jeden Tag automatisch zum konfigurierten Zeitpunkt ein und beginnt mit der Messung. Das Gerät schaltet sich automatisch zum konfigurierten Zeitpunkt aus.
Nach dem automatischen Einschalten führt das Gerät einen Signaltest durch, bei dem es nach Signalen sucht. Findet Das Gerät Signale innerhalb der programmierten Zeit, beginnt es die Aufzeichnung und speichert die Messwerte. Falls keine Signale anliegen, schaltet sich das Gerät wieder aus. Im Rhythmus von 20 Minuten schaltet es sich immer wieder an und führt wiederum einen Signaltest durch. Nach 4 Stunden schaltet sich Das Gerät ganz aus. Der Signaltest dauert maximal 2 Minuten. Falls innerhalb von 2 Minuten keine Signale gefunden werden, schaltet sich das Gerät aus.
Um in der Software eine manuelle Messung einzustellen, wird im Messmodus als Gerätetypen manuelle Messung ausgewählt. Ist die manuelle Messung aktiviert, muss das Gerät einmalig konfiguriert werden. Um eine manuelle Messung zu starten, muss die Taste (8) 3 Sekunden lang gedrückt. Das Gerät schaltet sich ein. Nach der definierten Aufzeichnungsdauer schaltet sich das Gerät automatisch wieder aus. Bei einer manuellen Messung wird immer die Konfiguration der vorangegangenen Messung verwendet.

Nach dem manuellen Einschalten innerhalb der programmierten Zeit führt das Gerät einen Signaltest durch, bei dem es nach Signalen sucht. Findet das Gerät Signale, beginnt es die Aufzeichnung und speichert die Messwerte. Falls keine Signale anliegen, schaltet sich das Gerät wieder aus. Falls die manuelle Messung vor der automatischen Messung außerhalb der programmierten Zeit gestartet wird, hat die manuelle Messung Vorrang.

Wenn eine automatische Messung läuft, kann keine manuelle Aufzeichnung gestartet werden.

Das Gerät schaltet sich nach 5 Minuten automatisch aus, owenn keine Aktionen durchgeführt werden oder owenn bei einer automatischen Messung der konfigurierte Endzeitpunkt erreicht ist oder owenn bei einer manuellen Messung das Ende der Aufzeichnungsdauer erreicht ist.

Das Display schaltet sich zum Stromsparen nach 30 Sekunden aus. Während des Signaltests, der maximal 2 Minuten dauert, bleibt das Display an. Die Signalkontrolle dauert maximal 2 Minuten, findet das Gerät keine Signale, schaltet es sich ab. Die Analyseergebnisse der letzten Nacht werden angezeigt, wenn keine Sensoren eingesteckt sind.

Das Gerät wird mittels nur einer Bedieneinrichtung (8) bedient. Über diese lassen sich alle Menuepunkte und Betriebsarten auswählen.

## Patentansprüche

1. Vorrichtung zum Messen von Biosignalen zur Erkennung schlafbezogener Atmungsstörungen mit einem Gehäuse (1), welches eine Oberseite (3) und eine Unterseite (4) aufweist,
- mit mindestens einer ersten Schnittstelle (6a, 6b) im Bereich des Gehäuses (1) eingerichtet zum Anschluss von Sensoreinrichtungen, und mit einer zweiten Schnittstelle (13) eingerichtet zur Verbindung mit einem PC,
- mit einer Anzeigeeinrichtung (7) im Bereich des Gehäuses (1), eingerichtet zur Anzeige von Messwerten,
- mit einer Bedieneinrichtung (8) eingereichtet zur Funktionssteuerung der Vorrichtung
- mit einer Befestigungseinrichtung (2) eingereichtet zum Befestigen des Gehäuses (1) an einem Patienten,
wobei die zumindest eine erste Schnittstelle (6), die Anzeigeeinrichtung (7) und die Bedieneinrichtung (8) im Bereich der Oberseite (3) oder von Seitenteilbereichen (5) des Gehäuses (1) angeordnet sind, welche die Oberseite und die Unterseite verbinden, wobei die Vorrichtung zur Verarbeitung von Messinformationen von den Sensoreinrichtungen eine Recheneinheit aufweist, die in dem Gehäuse (1) zur Verarbeitung der Messsignale angeordnet ist, und einen Speicher aufweist, der mit der Recheneinheit zum Anzeigen und Speichern der Messinformationen in Verbindung steht, wobei die Sensoreinrichtungen ein Pulsoximetriesensor (15') und eine Staudruck-Nasenbrille (14') zur Erfassung des Atemflusses bzw. der Sauerstoffsättigung des Blutes und der Pulsfrequenz des Patienten sind, wobei die Recheneinheit und der Speicher dazu eingerichtet sind, die Messdaten während einer Messung zu speichern und parallel zumindest teilweise auf dem integrierten Display anzuzeigen, wobei die zweite Schnittstelle (13) dazu eingerichtet ist, die im Speicher gespeicherten Daten auf einen PC (17) zu übertragen, wo mittels einer PC-Software die Signale sicht- und editierbar sind, **dadurch gekennzeichnet, dass** die Vorrichtung dazu eingerichtet ist, über die zweite Schnittstelle (13) mittels eines PC für eine automatische Messung derart konfiguriert zu werden, dass sich die Vorrichtung automatisch zu einem konfigurierten Zeitpunkt einschaltet und mit der Messung beginnt.

2. Vorrichtung nach Anspruch 1, **dadurch gekennzeichnet, dass** das Gehäuse lösbar mit der Befestigungseinrichtung (2) verbunden ist.

3. Vorrichtung nach Anspruch 1, **dadurch gekennzeichnet, dass** die Befestigungseinrichtung (2) eine Aufnahmefläche (11) für das Gehäuse aufweist und von der Aufnahmefläche Laschen (12) abgehen, die der Befestigung an einem Körperteil dienen.

4. Vorrichtung nach einem der Ansprüche 1 bis 3, **dadurch gekennzeichnet, dass** die zweite Schnittstelle im Bereich der Unterseite (4) des Gehäuses angeordnet und als eine USB-Schnittstelle (13) ausgebildet ist.

5. Vorrichtung nach Anspruch 4, **dadurch gekennzeichnet, dass** die USB-Schnittstelle (13) nach einer Montage des Gehäuses im Bereich der Befestigungseinrichtung (2) von der Befestigungseinrichtung (2) abgedeckt ist.

6. Vorrichtung nach Anspruch 4, **dadurch gekennzeichnet, dass** die USB-Schnittstelle (13) derart angeordnet und isoliert ist, dass im Betrieb der Vorrichtung ein Kontakt zwischen einem Nutzer und der USB-Schnittstelle ausgeschlossen ist.

7. Vorrichtung nach Anspruch 1, **dadurch gekennzeichnet, dass** die Vorrichtung mittels nur einer Bedieneinrichtung (8) bedient werden kann, die als druckbare Taste ausgeführt ist, die dem Ein- und Ausschalten sowie der Navigation im Gerätemenü dient.

8. Vorrichtung nach Anspruch 1 und 2, **dadurch gekennzeichnet, dass** das Gehäuse Befestigungselemente (9) aufweist, die der Befestigung des Gehäuses an der Befestigungseinrichtung (2) dienen.

9. Vorrichtung nach Anspruch 8, **dadurch gekennzeichnet, dass** die Befestigungselemente (9) als Zapfen im Seitenteilbereich des Gehäuses ausgebildet sind und die Zapfen in einem montierten Zustand in Ausnehmungen (10) der Befestigungseinrichtung (2) eingreifen.

10. Vorrichtung nach Anspruch 8, **dadurch gekennzeichnet, dass** zapfenförmige Befestigungselemente (9) im Bereich der Befestigungseinrichtung (2) und korrespondierende Aufnahmen im Bereich des Gehäuses angeordnet sind.

## Claims

1. Device for measuring biosignals to detect sleep-associated breathing disorders, comprising a housing (1) which has an upper face (3) and a lower face (4),
- comprising at least one first interface (6a, 6b) in the region of the housing (1), set up for linking sensor means, and comprising a second interface (13) set up for connection to a PC,
- comprising a display means (7) in the region of the housing (1), set up to display measurement values,
- comprising an operating means (8) set up for controlling the operation of the device,
- comprising an attachment means (2) set up for attaching the housing (1) to a patient,
the at least one first interface (6), the display means (7) and the operating means (8) being arranged in the region of the upper face (3) or of lateral sub-regions (5) of the housing (1) which connect the upper face and the lower face, the device having an arithmetic unit for processing measurement information from the sensor means, which unit is arranged in the housing (1) to process the measurement signals, and having a memory which is connected to the arithmetic unit to display and store the measurement information, the sensor means being a pulse oximetry sensor (15') and a diagnostic nasal cannula (14') for detecting the respiratory flow or the oxygen saturation of the blood and the pulse rate of the patient, the arithmetic unit and the memory being set up to store the measurement data during a measurement and, in parallel, to display them at least in part on the integrated display, the second interface (13) being set up to transfer the data stored in the memory onto a PC (17) where the signals can be viewed and edited using a PC software, **characterised in that** the device is set up to be configured, via the second interface (13) by way of a PC, for an automatic measurement, in such a way that the device switches on and starts the measurement automatically at a set time.

2. Device according to claim 1, **characterised in that** the housing is detachably connected to the attachment means (2).

3. Device according to claim 1, **characterised in that** the attachment means (2) has a receiving surface (11) for the housing, and straps (12) for attachment to a body part proceed from the receiving surface.

4. Device according to any of claims 1 to 3, **characterised in that** the second interface is arranged in the region of the lower face (4) of the housing and is formed as a USB interface (13).

5. Device according to claim 4, **characterised in that** the USB interface (13) is covered by the attachment means (2) after the housing is mounted in the region of the attachment means (2).

6. Device according to claim 4, **characterised in that** the USB interface (13) is arranged and insulated in such a way that contact between the user and the USB interface is not possible during operation of the device.

7. Device according to claim 1, **characterised in that** the device can be operated by way of just one operating means (8), which is configured as a pressable button for switching on and off and for navigation in the appliance menu.

8. Device according to claims 1 and 2, **characterised in that** the housing has attachment elements (9) for attaching the housing to the attachment means (2).

9. Device according to claim 8, **characterised in that** the attachment elements (9) are formed as pins in the lateral sub-region of the housing, and in a mounted state the pins engage in recesses (10) in the attachment means (2).

10. Device according to claim 8, **characterised in that** pin-shaped attachment elements (9) are arranged in the region of the attachment means (2) and corresponding recesses are arranged in the region of the housing.

## Revendications

1. Dispositif servant à mesurer des signaux biologiques pour la détection de troubles respiratoires liés au sommeil, ledit dispositif comprenant un boîtier (1) qui présente une partie supérieure (3) et une partie inférieure (4),
- comprenant au moins une première interface (6a, 6b) placée dans la zone du boîtier (1) et agencée pour le raccordement de dispositifs à capteurs et comprenant une deuxième interface (13) agencée pour la connexion à un PC,
- comprenant un dispositif de visualisation (7) agencé dans la zone du boîtier (1) et servant à l'affichage de valeurs mesurées,
- comprenant un dispositif de commande (8) agencé pour assurer la commande des fonctions du dispositif,
- comprenant un dispositif de fixation (2) agencé pour fixer le boîtier (1) sur un patient,
où la première interface (6) au moins au nombre de un, le dispositif de visualisation (7) et le dispositif de commande (8) sont disposés dans la zone de la partie supérieure (3) ou dans des zones de parties latérales (5) du boîtier (1), qui assemblent la partie supérieure et la partie inférieure, où le dispositif servant au traitement d'informations mesurées provenant des dispositifs à capteurs présente une unité de calcul qui est disposée dans le boîtier (1) pour le traitement des signaux mesurés, et présente une mémoire qui communique avec l'unité de calcul pour la visualisation et le stockage en mémoire des informations mesurées, où les dispositifs à capteurs sont un capteur d'oxymètre de pouls (15') et des lunettes nasales à pression dynamique (14') servant à la détection du débit respiratoire ou de la saturation en oxygène contenu dans le sang, et à la détection de la fréquence de pulsations du patient, où l'unité de calcul et la mémoire sont agencées dans le but de stocker en mémoire, au cours d'une mesure, les données mesurées et, parallèlement, d'afficher au moins partiellement lesdites données sur l'écran intégré, où la deuxième interface (13) est agencée dans le but de transmettre à un PC (17) les données stockées dans la mémoire, où les signaux, grâce à un logiciel de PC, sont visibles et modifiables, **caractérisé en ce que** le dispositif est agencé dans le but d'être configuré pour une mesure automatique, au moyen d'un PC, via la deuxième interface (13), de manière telle que le dispositif se mette en marche automatiquement à un moment configuré et que la mesure commence.

2. Dispositif selon la revendication 1, **caractérisé en ce que** le boîtier est assemblé avec le dispositif de fixation (2), de manière détachable.

3. Dispositif selon la revendication 1, **caractérisé en ce que** le dispositif de fixation (2) présente une surface de logement (11) pour le boîtier, et des languettes (12) partent de la surface de logement, languettes qui servent à la fixation sur une partie du corps.

4. Dispositif selon l'une quelconque des revendications 1 à 3, **caractérisé en ce que** la deuxième interface est disposée dans la zone de la partie inférieure (4) du boîtier et conçue comme une interface USB (13).

5. Dispositif selon la revendication 4, **caractérisé en ce que** l'interface USB (13), après un montage du boîtier dans la zone du dispositif de fixation (2), est recouverte par le dispositif de fixation (2).

6. Dispositif selon la revendication 4, **caractérisé en ce que** l'interface USB (13) est disposée et isolée de manière telle, qu'au cours du fonctionnement du dispositif, un contact entre un utilisateur et l'interface USB soit exclu.

7. Dispositif selon la revendication 1, **caractérisé en ce que** le dispositif peut être actionné au moyen seulement d'un dispositif de commande (8) qui est conçu comme une touche qui peut être activée et qui sert à la mise en marche et à l'arrêt, ainsi qu'à la navigation dans le menu de l'appareil.

8. Dispositif selon la revendication 1 ou 2, **caractérisé en ce que** le boîtier présente des éléments de fixation (9) qui servent à la fixation du boîtier sur le dispositif de fixation (2).

9. Dispositif selon la revendication 8, **caractérisé en ce que** les éléments de fixation (9) sont conçus comme des tenons placés dans la zone de la partie latérale du boîtier, et les tenons, à l'état monté, s'engagent dans des creux (10) du dispositif de fixation (2).

10. Dispositif selon la revendication 8, **caractérisé en ce que** des éléments de fixation (9) en forme de tenons sont disposés dans la zone du dispositif de fixation (2), des logements correspondants étant disposés dans la zone du boîtier.
